# EUROPÄISCHE PATENTSCHRIFT

(11) **EP 1 323 391 B1**
(45) Veröffentlichungstag und Bekanntmachung des Hinweises auf die Patenterteilung: **11.03.2009**
(21) Anmeldenummer: 02026606.0
(22) Anmeldetag: 28.11.2002
(51) Int. Cl.: A61B 17/70

(54) **Verschlusseinrichtung zum Sichern eines Stabes in einem Aufnahmeteil insbesondere einer Polyaxial-Knochenschraube**
Locking device for securing a rod in a holder, especially of a polyaxial bone screw
Dispositif de verrouillage pour bloquer une tige dans un réceptacle, en particulier une vis osseuse polyaxiale

(30) Priorität: 28.12.2001 DE 10164323
(43) Veröffentlichungstag der Anmeldung: 02.07.2003
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- US-A- 5 443 467
- US-A- 5 672 176
- US-A- 5 797 911

## Beschreibung

Die Erfindung betrifft ein in der Wirbelsäulen- bzw. Unfallchirurgie zu verwendende Verschlußeinrichtung zum Sichern eines stabförmigen Elements in einem mit einem Schaft verbundenen Halteelement. Ferner betrifft die Erfindung ein Element mit einem Schaft und einem damit verbundenen Halteelement zum Verbinden mit einem Stab mit einer derartigen Verschlußeinrichtung, insbesondere eine Polyaxial-Knochenschraube.

Aus der WO 98/25534 ist eine Polyaxial-Knochenschraube bekannt, bei der der Kopf der Knochenschraube und der Stab unabhängig voneinander relativ zu dem Aufnahmeteil fixierbar sind. US-A- 5 443 467 offenbart eine Vorrichtung mit den Merkmalen des Oberbegriffs von Anspruch 4.

Aufgabe der Erfindung ist es, eine Verschlußeinrichtung der eingangs beschriebenen Art und ein Element mit einem Schaft und einem damit verbundenen Halteelement zu schaffen, welches eine verbesserte Sicherung gegen Lockerung oder Lösen der Verschlußelemente gewährleistet und gleichzeitig kompakt gebaut ist.

Diese Aufgabe wird durch die in Patentanspruch 1 gekennzeichnete Verschlußeinrichtung bzw. durch das in Patentanspruch 4 gekennzeichnete Element gelöst. Weiterbildungen der Erfindung sind in den Unteransprüchen gegeben.

Weitere Merkmale und Zweckmäßigkeiten der Erfindung ergeben sich aus der Beschreibung von Ausführungsbeispielen anhand der Figuren. Von den Figuren zeigen:
- Fig. 1:: eine Polyaxial-Knochenschraube nach einer Ausführungsform in geschnittener Explosionsdarstellung; und
- Fig. 2:: Die Polyaxial-Knochenschraube von Fig. 1 im Betriebszustand in geschnittener Darstellung.

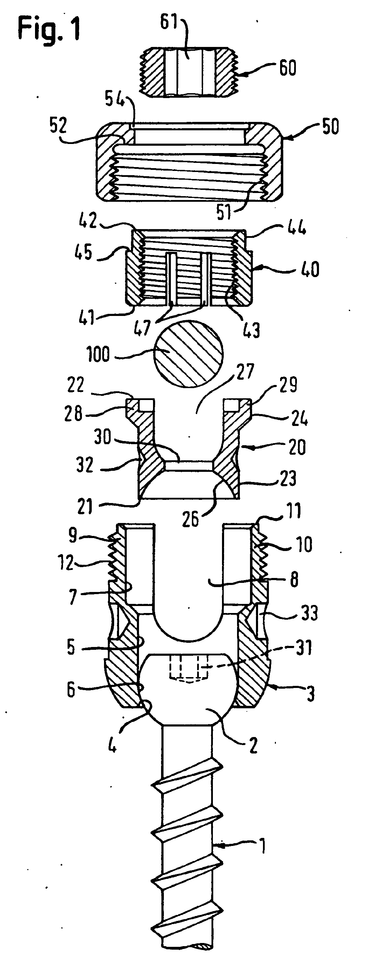

In der in Fig. 1 bis 2 gezeigten Ausführungsform ist das erfindungsgemäße Element als Polyaxialschraube ausgebildet, die ein Schraubenelement mit einem Gewindeschaft 1 mit einem Knochengewinde und einem kugelsegmentförmigen Kopf 2 aufweist, der mit einem Aufnahmeteil 3 verbunden ist. Das Aufnahmeteil 3 hat an seinem einen Ende eine axialsymmetrisch ausgerichtete erste Bohrung 4, deren Durchmesser größer als der des Gewindeabschnitts des Schafts 1 und kleiner als der des Kopfes 2 ist. Ferner weist das Aufnahmeteil 3 eine koaxiale zweite Bohrung 5 auf, die auf dem der ersten Bohrung 4 gegenüberliegenden Ende offen ist und deren Durchmesser so groß ist, daß das Schraubenelement durch das offene Ende mit seinem Gewindeabschnitt durch die erste Bohrung 4 hindurch und mit dem Kopf 2 bis zum Grund der zweiten Bohrung 5 führbar ist. Zwischen der ersten und der zweiten Bohrung ist ein kleiner koaxialer Abschnitt 6 vorgesehen, der unmittelbar an die erste Bohrung 4 angrenzt und zum offenen Ende hin sphärisch ausgebildet ist, wobei der Radius im wesentlichen gleich dem kugelsegmentförmigen Abschnitts des Kopfs 2 ist. Die zweite Bohrung 5 erweitert sich gegen das der ersten Bohrung 4 abgewandte Ende wodurch ein Bereich 7 mit einem größeren Durchmesser gebildet ist.

Das Aufnahmeteil 3 weist ferner eine zur Mitte des Teiles symmetrisch angeordnete U-förmige Ausnehmung 8 zur Aufnahme eines Stabs 100 auf, deren Grund zur ersten Bohrung 4 hin gerichtet ist und durch die zwei freie Schenkel 9, 10 gebildet sind, deren freies Ende 11 den oberen Rand des Aufnahmeteils 3 bildet. In einem Bereich angrenzend an das freie Ende 11 weisen die Schenkel 9, 10 ein Außengewinde 12 auf.

Es ist ferner ein Druckelement 20 mit einem ersten Ende 21, das in einem in das Aufnahmeteil 3 eingesetztem Zustand dem Kopf 2 zugewandt ist, und einem dem ersten Ende gegenüberliegenden zweiten Ende 22 vorgesehen. Das Druckelement weist angrenzend an das erste Ende 21 einen ersten zylindrischen Bereich 23 auf mit einem Durchmesser, der etwas kleiner als der der Bohrung 5 angrenzend an den spärischen Abschnitt 6 ist, so daß das Druckelement in dem Bereich der Bohrung 5 gleiten kann, also zu dem Kopf 2 hin verschiebbar ist. Der erste zylindrische Bereich 23 geht mit kegeliger Außenfläche über in einen zweiten zylindrischen Bereich 24, der sich bis zum zweiten Ende 22 erstreckt und einen Durchmesser aufweist, der etwas kleiner ist als der des Bereichs 7 des Aufnahmeteils 3 derart, daß das Druckelement 20 mit seinem zweiten zylindrischen Bereich 24 in dem Bereich 7 des Aufnahmeteils 3 gleiten kann.

Das Druckelement weist ferner an seinem ersten Ende 21 eine sich zu dem Ende hin erweiternde kugelsegmentförmige Ausnehmung 26 auf, deren Kugelradius so gewählt ist, dass er in einem in das Aufnahmeteil eingesetzten Zustand den Kopf 2 teilweise umgreift.

Ferner ist an dem Druckelement 20 an dem der kugelsegmentförmigen Ausnehmung 26 gegenüberliegenden zweiten Ende 22 eine U-förmige Ausnehmung 27 auf, wobei die Abmessungen der U-förmigen Ausnehmungen des Druckelements so bemessen sind, in dem in das Aufnahmeteil 3 eingesetztem Zustand die U-förmige Ausnehmung 8 des Aufnahmeteils und die U-förmige Ausnehmung 27 des Druckelements einen Kanal bilden, in den der Stab 100 eingelegt werden kann. Die in Richtung der Zylinderachse des Aufnahmeteils 3 gesehene Tiefe der U-förmigen Ausnehmung 27 ist größer als der Durchmesser des aufzunehmenden Stabs 100, so dass das Druckelement 20 mit seitlichen Schenkeln 28, 29 über den eingelegten Stab 100 nach oben hervorsteht. Das Druckelement weist ferner eine zentrale Bohrung 30 auf, die sich durch dieses hindurch erstreckt und deren Durchmesser gerade so groß bemessen ist, dass ein Schraubwerkzeug zum Ineingriffbringen mit einer in dem Kopf 2 vorgesehenen Ausnehmung 31 hindurchführbar ist. In seinem ersten zylindrischen Abschnitt 23 sind ferner umfangsseitig an dem Druckelement Senkbohrungen 32 vorgesehen, die mit entsprechenden Kröpfbohrungen 33 an dem Aufnahmeteil 3 zum leichten Halten des Drukkelements in der Stellung, in der der Kanal gebildet wird, zusammenwirken.

Ferner ist ein hülsenartiges Element 40 mit zylindrischer Form mit einem ersten Ende 41 und einem diesem gegenüberliegenden zweiten Ende 42 vorgesehen, wobei der Außendurchmesser des Elements 40 so groß, daß das Element 40 in dem Bereich 7 des Aufnahmeteils vom freien Ende 11 her einsetzbar ist und darin gleiten kann. Das hülsenartige Element 40 weist eine zentrale Bohrung mit einem Innengewinde 43 zur Aufnahme einer Innenschraube 60 auf. Angrenzend an das zweite Ende ist eine ringförmige Ausnehmung 44 in der Außenwand vorgesehen, durch die eine Schulter 45 gebildet ist. In der Ausführungsform gemäß Fig. 1 erstrecken sich in axialer Richtung vom ersten Ende 41 des Elements 40 durch seine Wand bis auf die Höhe der Schulter 45 eine Mehrzahl von Schlitzen 47. Diese bewirken, daß das Element 40 hauptsächlich einerseits in horizontaler Richtung elastisch ist und in vertikaler Richtung als ein stabiler Druckring wirkt. Die axiale Länge des Elements 40 ist derart bemessen, daß das Element, wenn es zusammen mit dem Schraubenkopf 2 und dem Druckelement 20 in das Aufnahmeteil eingesetzt ist und mit seinem ersten Ende 41 auf den Schenkeln 28, 29 des Druckelements aufliegt, mit seiner Schulter 45 über den Rand 11, des Aufnahmeteils etwas hervorsteht.

Zum Fixieren des Elements 40 ist eine die Schenkel 9, 10 von außen umfassende Außenmutter 50 vorgesehen, deren Innengewinde 51 mit dem Außengewinde 12 der Schenkel 9, 10 des Aufnahmeteils 3 zusammenwirkt. Die Außenmutter 50 weist an einem Ende einen radial nach innen gerichteten Ansatz 52 auf, der in aufgeschraubtem Zustand der Außenmutter 50 auf die Schulter 45 des Elements 40 drückt. Die axiale Länge der Außenmutter 50 ist ferner so bemessen, daß in aufgeschraubtem Zustand die Außenmutter gerade nicht auf den eingelegten Stab 100 drückt, so daß dieser in dem Kanal verschiebbar ist.

Die Innenschraube 60 ist in das hülsenartige Element 40 einschraubbar. Hierzu weist sie eine Ausnehmung 61 zum Ineingriffbringen mit einem Eindrehwerkzeug auf.

Im Betrieb wird zuerst das Schraubenelement in das Aufnahmeteil 3 eingeführt, bis der Kopf 2 an dem sphärischen Abschnitt 6 anliegt. Dann wird das Druckelement 20 eingesetzt und über das Zusammenwirken der Senk- und Kröpfbohrungen 32 so gehalten, daß die U-förmige Ausnehmung 8 des Aufnahmeteils und die U-förmige Ausnehmung 27 des Druckelements 20 aufeinander zuliegen kommen. Das Druckelement ist somit gegen Herausfallen gesichert. In diesem Zustand schraubt der Chirurg das Schraubenelement über die Ausnehmung 31 in den Knochen ein. Dann wird der Stab 100 eingelegt und das hülsenartige Element 40 mit bereits eingeschraubter Innenschraube 60 vom freien Ende 11 der Schenkel her zwischen die Schenkel 9, 10 einsetzt. Dabei ist die Innenschraube 60 nur soweit in das Element 40 eingeschraubt, daß ihre dem Stab zugewandte Seite den Stab noch nicht berührt. Anschließend wird die Außenmutter 50 aufgeschraubt. Solange ihr nach innen gerichteter Ansatz 52 noch nicht auf der Schulter 45 des Elements 40 aufliegt, ist der Kopf 2 des Schraubenelements noch nicht fixiert. Erst bei weiterem Aufschrauben der Außenmutter 50 drückt der Ansatz 52 auf die Schulter 45 und somit auf das Element 40, was seinerseits wieder mit seinem ersten Ende 41 eine Kraft auf die Schenkel 28, 29 des Druckelements 20 ausübt wodurch dieses gegen den Kopf 2 drückt und diesen in seiner Stellung fixiert. Weil die Schenkel 28, 29 des Druckelements über den eingelegten Stab 100 hervorstehen, ist dieser weiterhin verschiebbar. Anschließend wird der Stab durch tieferes Einschrauben der Innenschraube 60 bis diese auf den Stab 100 einwirkt fixiert.w

Beim Aufschrauben der Außenmutter wirkt auf die Schenkel 9, 10 des Aufnahmeteils eine Kraft, die diese leicht nach innen drückt. Somit wird das geschlitzte Element 40 etwas zusammengedrückt. Zum endfesten Einschrauben der Innenschraube 60 zum Fixieren des Stabs wirkt eine radial nach außen gerichtete Kraftkomponente über die Gewindeflanken, die das Element 40 dann aufweitet. Dadurch verspreizt sich das Element 40 gegen die Schenkel 9, 10 des Aufnahmeteils und das Gewinde 12, das die Außenmutter hält, was ein Lockern oder gar Lösen des Verschlußmechanismus wirkungsvoll verhindert.

Der beschriebene Verschlußmechanismus gewährleistet außerdem, daß das Druckelement 20 nur Kräfte in axialer Richtung, nicht aber in radialer Richtung erfährt, so daß seine Funktion des Fixierens des Kopfes auch nach Einschrauben der Innenschraube nicht beeinträchtigt ist.

In einer Abwandlung ist das Innengewinde 43 des Elements 40 als Linksgewinde ausgebildet, während das Außengewinde 12 der Schenkel des Aufnahmeteils 3 und das Gewinde der Außenmutter als Rechtsgewinde ausgebildet sind.

Anstelle der oben beschriebenen Ausführungen, bei denen der Schaft 1 mit einem Knochengewinde ausgebildet ist, kann der Schaft auch als Haken wie er in der Wirbelsäulenchirurgie zum Einhaken hinter Knochenvorsprüngen der Wirbelsäule verwendet wird, ausgebildet sein.

In einer weiteren Abwandlung der polyaxialen Ausführungsformen ist anstelle des Gewindeschaftes 1 oder des Hakens eine Stange oder ein stabförmiges Element vorgesehen, das an beiden Enden einen kugelsegmentförmigen Kopf hat, der mit einem Aufnahmeteil der beschriebenen Art verbunden ist. Damit kann ein solches Element als Verbindungselement zwischen zwei Stäben 100 verwendet werden.

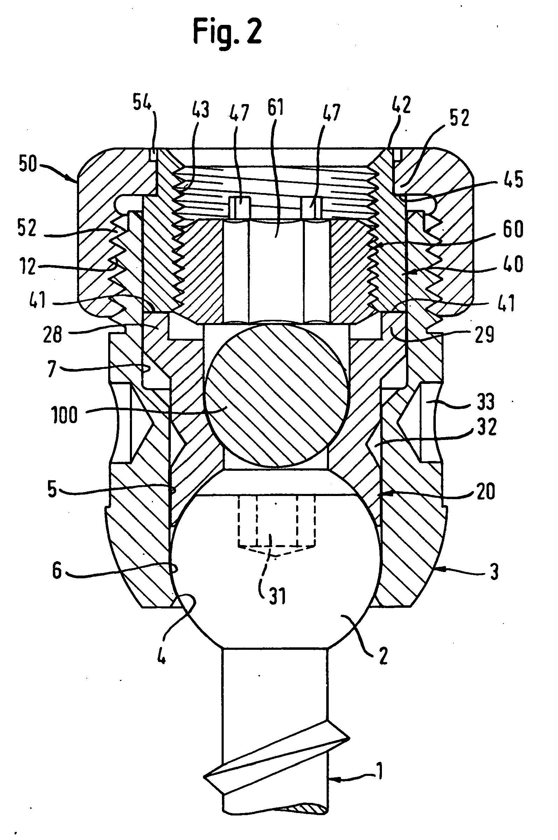

In einer weiteren Abwandlung ist das hülsenartige Element 40 mit der Außenmutter drehbar verbunden. Hierzu ist das zweite Ende 42 des Elements 40 radial nach außen umgebogen (in den Figuren nicht dargestellt), so daß es in eine in Fig. 1 und 2 gezeigte ringförmige Ausnehmung 54 an der Oberseite der Außenmutter 50 eingreift. Im Betrieb wird dann die Außenmutter mit dem drehbar verbundenen Element 40 als Einheit auf die Schenkel aufgesetzt und die Außenmutter aufgeschraubt.

Die erfindungsgemäße Verschlußeinrichtung ist auch bei einer Monoaxial-Knochenschraube anwendbar. In diesem Fall wirkt das hülsenartige Element 40 nicht auf ein Druckelement, sondern wirkt zusätzlich zu der Innenschraube direkt auf den Stab ein. Hierzu ist das Element 40 von seiner axialen Länge her so bemessen, daß bei aufgeschraubter Außenmutter durch diese auf den Stab gedrückt wird. Beim Einschrauben der Innenschraube erfolgt wie bei der Ausführungsform der Polyaxialschraube ein Aufspreizen des Elements 40, wodurch ein Lockern oder gar Lösen der Stabklemmung zuverlässig verhindert wird.

## Patentansprüche

1. Verschlußeinrichtung zum Sichern eines stabförmigen Elements in einem mit einem Schaft (1) verbundenen Halteelement (3) zum Einsatz in der Wirbelsäulen oder Unfallchirurgie, mit
einem Element (40) mit zylindrischer Außenfläche und mit einem Außendurchmesser, der so bemessen ist daß das Element zwischen zwei freie Schenkel (9, 10) des Halteelements (3) einschiebbar ist, einer zentralen Bohrung mit einem Innengewinde (43) zur Aufnahme einer Innenschraube (60) und mit wenigstens einem sich von einem Ende über eine vorbestimmte Länge in der Zylinderwand erstreckenden Schlitz (47), und
mit einer mit einem Außengewinde (12) der freien Schenkel (9, 10) zusammenwirkenden Außenmutter (50), die in aufgeschraubtem Zustand das Element (40) zwischen den Schenkeln (9, 10)hält.

2. Verschlußeinrichtung nach Anspruch 1, **dadurch gekennzeichnet, daß** das Element (40) angrenzend an sein eines Ende (42) eine Aussparung (44) aufweist, wodurch eine Schulter (45) gebildet ist, die mit einem entsprechenden Ansatz (52) an der Außenmutter (50) zusammenwirkt.

3. Verschlußeinrichtung nach einem der Ansprüche 1 oder 2, **dadurch gekennzeichnet, daß** das Element (40) eine Mehrzahl von sich von einem freien Ende (41) in axialer Richtung auf eine vorbestimmte Länge erstreckende Schlitze (47) aufweist.

4. Element mit einem Schaft und einem damit verbundenen Halteelement zum Verbinden mit einem Stab, wobei das Halteelement (3) eine einen U-förmigen Querschnitt aufweisende Ausnehmung (8) zur Aufnahme des Stabes (100) mit zwei an einem Ende freien Schenkeln (9, 10) und einem Außengewinde (12) an den freien Schenkeln aufweist, und
mit einer mit dem Außengewinde (12) zusammenwirkenden Außenmutter (50),
mit einen zwischen die Schenkel (9, 10) einsetzbaren Element (40) mit zylindrischer Außenfläche **dadurch gekennzeichnet, daß** das einsetzbare Element, einen Außendurchmesser anfweist, der so bemessen ist, daß das einsetzbar Element (40) zwischen den Schenkeln (9, 10) in axialer Richtung verschiebbar ist, und mit einem Innengewinde (43) zur Aufnahme einer Innenschraube (60) sowie mit wenigstens einem sich von einem Ende über eine vorbestimmte Länge in der Zylinderwand erstreckenden Schlitz (47) versehen ist.

5. Element nach Anspruch 4, **dadurch gekennzeichnet, daß** das einsetzbare Element (40) angrenzend an sein eines Ende (42) eine Aussparung (44) aufweist, wodurch eine Schulter (45) gebildet ist, die mit einem entsprechenden Ansatz (52) an der Außenmutter (50) zusammenwirkt.

6. Element nach Anspruch 4 oder 5, **dadurch gekennzeichnet, daß** das einsetzbare Element (40) eine Mehrzahl von sich von einem freien Ende (41) in axialer Richtung auf eine vorbestimmte Länge erstreckende Schlitze (47) aufweist.

7. Element nach einem der Ansprüche 4 bis 6, **dadurch gekennzeichnet, daß** der Schaft (1) und das Halteelement (3) polyaxial verbunden sind.

8. Element nach Anspruch 7, **dadurch gekennzeichnet, daß** in dem Halteelement (3) ein Druckelement (20) vorgesehen ist, auf welches das einsetzbare Element (40) zum Fixieren der Winkelstellung des Schafts (1) einwirkt.

9. Element nach einem der Ansprüche 4 bis 8, **dadurch gekennzeichnet, daß** der Stab (100) über die Innenschraube (60) fixierbar ist.

## Claims

1. Locking device for securing a rod-shaped element in a holding element (3) connected to a shaft (1), for use in spinal surgery or accident and emergency surgery, having
an element (40) with a cylindrical outer surface and with an external diameter which is dimensioned such that the element can be pushed in between two free legs (9, 10) of the holding element (3), a central bore with an internal thread (43) for receiving an inner screw (60) and with at least one slit (47) which extends from one end over a predetermined length in the cylinder wall, and
having an outer nut (50) which interacts with an external thread (12) of the free legs (9, 10) and, when screwed on, holds the element (40) between the legs (9, 10).

2. Locking device according to Claim 1, **characterized in that** one end (42) of the element (40) is adjoined by a recess (44), and this forms a shoulder (45) which interacts with a corresponding projection (52) on the outer nut (50).

3. Locking device according to either of Claims 1 and 2, **characterized in that** the element (40) has a plurality of slits (47) extending from one free end (41) in the axial direction over a predetermined length.

4. Element with a shaft and a holding element which is connected to the latter and which serves for connection to a rod, wherein the holding element (3) has a recess (8) with a U-shaped cross section for receiving the rod (100) and with two legs (9, 10) that are free at one end and an external thread (12) on the free legs, and
with an outer nut (50) which interacts with the external thread (12),
with an element (40) which can be inserted between the legs (9, 10) and has a cylindrical outer surface, **characterized in that** the insertable element has an external diameter which is dimensioned such that the insertable element (40) can be displaced between the legs (9, 10) in the axial direction, and is provided with an internal thread (43) for receiving an inner screw (60) and with at least one slit (47) which extends from one end over a predetermined length in the cylinder wall.

5. Element according to Claim 4, **characterized in that** one end (42) of the insertable element (40) is adjoined by a recess (44), and this forms a shoulder (45) which interacts with a corresponding projection (52) on the outer nut (50).

6. Element according to Claim 4 or 5, **characterized in that** the insertable element (40) has a plurality of slits (47) extending from one free end (41) in the axial direction over a predetermined length.

7. Element according to one of Claims 4 to 6, **characterized in that** the shaft (1) and the holding element (3) are connected polyaxially.

8. Element according to Claim 7, **characterized in that** a pressure element (20) is provided in the holding element (3), the insertable element (40) acting on said pressure element in order to fix the angular position of the shaft (1).

9. Element according to one of Claims 4 to 8, **characterized in that** the rod (100) can be fixed by means of the inner screw (60).

## Revendications

1. Dispositif de verrouillage pour bloquer un élément en forme de barre dans un élément de maintien (3) relié à une tige (1), pour utilisation dans les colonnes vertébrales ou la chirurgie des accidents, avec
un élément (40) à surface extérieure cylindrique et avec un diamètre extérieur de dimensions telles que l'élément puisse être inséré entre deux branches (9, 10) libres de l'élément de maintien (3), un perçage central avec un taraudage (43) pour recevoir une vis intérieure (60), et avec au moins une fente (47), s'étendant depuis une extrémité, sur une longueur prédéterminée, dans la paroi cylindrique, et
avec un écrou extérieur (50), coopérant avec un filetage extérieur (12) des branches (9, 10) libres, maintenant, à l'état vissé, l'élément (40) entre les branches (9, 10).

2. Dispositif de verrouillage selon la revendication 1, **caractérisé en ce que** l'élément (40) présente, en limite à une extrémité (42) de ses extrémités, un creusement (44), faisant qu'est formé un épaulement (45) coopérant avec un appendice (52) correspondant, situé sur l'écrou extérieur (50).

3. Dispositif de verrouillage selon l'une des revendications 1 ou 2, **caractérisé en ce que** l'élément (40) présente une pluralité de fentes (47) s'étendant à partir d'une extrémité (41) libre, en direction axiale, sur une longueur prédéterminée.

4. Elément avec une tige et un élément de maintien lui étant relié, pour liaison à une barre, l'élément de maintien (3) présentant un évidement (8) présentant une section transversale en fore de U, pour recevoir la barre (100), avec deux branches (9, 10) libres à une extrémité et un filetage extérieur (12) sur les branches libres, et
avec un écrou extérieur (50), coopérant avec le filetage extérieur (12),
avec un élément (40) à surface extérieure cylindrique, susceptible d'être inséré entre les branches (9, 10), **caractérisé en ce que** l'élément susceptible d'être inséré présente un diamètre extérieur de dimensions telles que l'élément susceptible d'être inséré soit déplaçable en direction axiale entre les branches (9, 10), et est muni d'un taraudage (43) pour recevoir une vis intérieure (60), ainsi que d'au moins une fente (47), s'étendant depuis une extrémité, sur une longueur prédéterminée, dans la paroi cylindrique.

5. Elément selon la revendication 4, **caractérisé en ce que** l'élément susceptible (40) d'être inséré présente, en limite à une extrémité (42) de ses extrémités, un creusement (44), faisant qu'est formé un épaulement (45) coopérant avec un appendice (52) correspondant, situé sur l'écrou extérieur (50).

6. Elément selon la revendication 4 ou 5, **caractérisé en ce que** l'élément susceptible (40) d'être inséré présente une pluralité de fentes (47) s'étendant à partir d'une extrémité (41) libre, en direction axiale, sur une longueur prédéterminée.

7. Elément selon l'une des revendications 4 à 6, **caractérisé en ce que** la tige (1) et l'élément de maintien (3) sont reliés de manière polyaxiale.

8. Elément selon la revendication 7, **caractérisé en ce que,** dans l'élément de maintien (3), est prévu un élément de pressage (20), sur lequel agit l'élément susceptible (40) d'être inséré, pour fixer la position angulaire de la tige (1).

9. Elément selon l'une des revendications 4 à 8, **caractérisé en ce que** la barre (100) est susceptible d'être fixée par l'intermédiaire de la vis intérieure (60).
